# EUROPEAN PATENT SPECIFICATION

(11) **EP 0 571 044 B1**
(45) Date of publication and mention of the grant of the patent: **09.04.1997**
(21) Application number: 93201427.7
(22) Date of filing: 18.05.1993
(51) Int. Cl.: C07C 67/54, C07C 69/54

(54) **Recovery of carboxylate ester reaction products**
Wiedergewinnung von Carbonsäureesterreaktionsprodukten
Récupération de produits de réaction d'esters carboxyliques

(30) Priority: 20.05.1992 EP 92201452
(43) Date of publication of application: 24.11.1993
(73) Proprietor: SHELL INTERNATIONALE RESEARCH MAATSCHAPPIJ B.V., 2596 HR Den Haag (NL)
(72) Inventor: Van Der Beek, Douwe Christiaan, NL-2596 HR The Hague (NL); De Blank, Peter Bastiaan, NL-2596 HR The Hague (NL); Jhalaria, Kishor, Belapur Rd., Thane 400 601 Maharashtra (IN)

(56) References cited:
- US-A- 4 070 254
- US-A- 4 518 462

## Description

This invention relates to a process for recovery of a carboxylate ester reaction product from a reaction zone by withdrawal and fractionation of a reaction mixture comprising said carboxylate ester in admixture with at least one azeotrope-forming alcohol precursor as well as further impurities. This invention more particularly relates to the recovery of a purified carboxylate ester, preferably an alkyl ester of acrylic or methacrylic acid, from a reaction effluent of a continuous production process, which comprises the carboxylate ester product in admixture with the corresponding alcohol forming an azeotrope with the ester, as well as further impurities which may or may not form azeotropes with the carboxylate ester and/or the alcohol.

Various production processes for carboxylate esters are effected using an alcohol precursor to the carboxylate ester at a concentration in the reaction zone in excess of the required stoichiometric amount. For example, in equilibrium reactions the reaction may thus be driven to near completion. Or, in catalysed reactions the conversion rate in terms of product produced per unit of catalyst and time may be increased. The alcohol precursor in excess of the required stoichiometric amount may be discarded, if it is cheap, but preferably is recycled to the reaction zone in a continuous or semi-continuous operation mode of the process. In any case it must be separated from the reaction product, for example by fractionation.

For reaction mixtures comprising an admixture of the carboxylate ester reaction product and the excess alcohol precursor, which exist in liquid form under the conditions of temperature and pressure applied, the fractionation conveniently utilises the different volatilities of the constituents to be separated, for example by distillation. This will present no problem for reaction mixtures of zeotropic nature, in which the constituents have discrete volatilities. Also topping and/or tailing of the reaction product and/or the recycled alcohol precursor from light end and/or heavy end contaminants can then be effected using standard chemical technology.

However, if the carboxylate ester reaction product and alcohol precursor form an azeotrope, this azeotrope will be separated due to its higher or, occasionally, lower volatility than that of the carboxylate ester reaction product. Since containing part of the reaction product, this azeotrope will usually be recycled to the reaction for economic reasons. In a continuous process, when recycling part of the reaction effluent, provisions should be effected for avoiding any build-up of contaminants, such as originating from the reactants, from side reactions or from catalyst residue. Accordingly, bleed streams containing such contaminants should be withdrawn from the process. Heavy ends having lower volatility and light ends having higher volatility than the reaction product or azeotrope can be cut off by flashing or as bleed streams and/or distillation overheads or bottoms at a suitable location, preferably where the highest concentrations of the contaminants concerned occur using conventional methods. These topping or tailing steps will not remove any intermediate contaminants, which have a volatility in between the volatilities of the azeotrope and the reaction product, or which form further azeotropes with the reaction product in this volatility range.

US-A-4280010 discloses a continuous process for the production of alkyl acrylates by reaction of acrylic acid with an excess of a lower alkanol, wherein the organic phase of the product stream comprising acrylate ester and precursor alcohol is fed to a distillation column for fractionation into an azeotrope stream of alcohol and ester over the top of the column, which stream is being recycled to the reactor, and a stream of crude ester product over the bottom of the column, which is subjected to further purification by distillation until pure. Heavy ends are removed from the system by a bleed stream from the reactor, whereas contaminants boiling between the ester/alcohol azeotrope and the pure ester apparently are removed in the final purification step.

US-A-4250328 discloses the production of methyl isobutyrate or methacrylate by a direct esterification process, wherein an excess of isobutyric or methacrylic acid precursor is used in the reaction zone for shifting the reaction equilibrium and minimising the amount of alcohol in the reaction effluent in an attempt to overcome the problems associated with azeotrope formation. However, this method is not applicable to processes, such as transesterifications and carbonylations, using other precursors than the free acid; leads to methacrylic acid losses due to required bleed streams; and may lead to polymerization problems in the methacrylic acid recycle stream.

Further processes aiming at the separation of components having close volatilities are known, for example from US-A-4070254, US-A-4518462 and EP-A-390577, which add an extraneous component to the mixture for forming an azeotrope with the impurities to be removed, which is more easily separated from the product to be purified.

Due to azeotrope formation, the known recovery processes have in common, that the separated stream containing the excess of alcohol precursor also comprises part of the carboxylate ester product. In practice, the concentration of carboxylate ester reaction product in the azeotrope stream will be higher even than its azeotropic concentration. Firstly, in a commercially operated distillation column the theoretical degree of separation can only be approximated using economically acceptable equipment. Secondly, as purification of the carboxylate ester product is the primary target of the recovery process, the distillation will preferably be operated such that the most effective separation regarding the carboxylate ester is achieved also in respect of further impurities present in the reaction effluent, which have a volatility in the range between the volatilities of the ester and the azeotrope. Therefore, in case the azeotrope has a volatility higher than that of the pure carboxylate ester the reaction effluent is preferably cut at a volatility just above the volatility of the pure carboxylate ester to achieve a fractionation into a product stream substantially free of lighter impurities and an azeotrope stream comprising all of the azeotrope forming alcohol precursor and intermediate impurities as well as a significant amount of the carboxylate ester. Since being recycled to the reaction zone, in principle this part of the carboxylate ester product is not lost. However, the presence of the intermediate impurities will lead to an undesired accumulation in a continuous process. This should be avoided by bleeding part of the recycle stream or by application of a further fractionation step into a substantially pure azeotrope for recycle and a bottoms containing the intermediate impurities for disposal.

After separation of the substantially pure azeotrope, this bottoms stream comprises some of the carboxylate ester product as its major constituent, which is disposed in view of its content of intermediate impurities with consequent reduction of the total yield of the carboxylate ester production process. Moreover, some impurities, such as water, in this bottoms stream may cause miscibility problems.

The present invention aims at solving these problems by providing a recovery process, which avoids the use of valuable reaction product as a carrier for the intermediate impurities to be bled from the process.

Accordingly, it is provided a recovery process as indicated above, which process comprises the steps of:
a) fractionating the reaction mixture into a carboxylate ester product-containing stream and a first azeotrope stream;
b) adding sufficiently additional azeotrope-forming alcohol precursor to the first azeotrope stream obtained by step (a) for providing a total concentration of the alcohol in excess of its azeotropic concentration; and
c) fractionating the alcohol-enriched stream obtained by step (b) into a second azeotrope stream for recycle to the reaction zone and a first residue stream for disposal.

By the process of the invention, it is achieved that the first residue stream containing intermediate impurities to be withdrawn from the carboxylate ester production process comprises the alcohol precursor as its major carrier component. Thus, an amount of cheaper alcohol precursor rather than of valuable ester product is discarded with consequent increase of the total yield of product. Moreover, alcohols generally having more versatile solvent properties than esters, any miscibility problems with aqueous constituents of the first residue stream are avoided.

Also, the present recovery method is very conveniently incorporated into a carboxylate ester production process. Part of the additional azeotrope-forming alcohol precursor added in step (b) will be recycled to the reaction zone due to a relative increase of the volume of the azeotrope stream obtained in step (c) and will serve as (part of) the feed of the carboxylate ester production process. The specific amount can readily be compensated by a corresponding decrease of the regular alcohol precursor feed. Moreover, the present method does not introduce any extraneous component, so that no complication of the purification steps occurs.

The present recovery method may be applied to reaction effluents from various carboxylate ester production processes, which use as one of the precursors an alcohol forming an azeotrope with the carboxylate ester product. Such processes typically include direct esterifications of carboxylic acids with alcohols, transesterifications of carboxylate esters with different alcohols, and carbonylations of ethylenically and/or acetylenically unsaturated compounds in the presence of alcohols.

Preferably, such processes are conducted employing a stoichiometric excess of alcohol precursor in the reaction zone, for example for shifting the equilibrium or increasing the catalyst efficiency. However, also when employing equivalent alcohol precursor feeds, the reaction effluent may contain remaining alcohol precursor due to incompleteness of the reaction and may then be subjected to the present recovery method. Since such effluents will also contain corresponding unreacted amounts of the other precursors complicating the purification steps, the latter operation mode is not preferred.

As typical examples of pairs of carboxylate ester and corresponding alcohol mention can be made of methanol/methyl acetate, methanol/methyl isobutyrate, methanol/methyl acrylate, methanol/methyl methacrylate, n-butanol/n-butyl formate, and n-pentanol/n-pentyl formate, all forming binary azeotropes. The present recovery method can also be used, if the carboxylate ester and corresponding alcohol together with further component(s) present in the reaction effluent form ternary or higher azeotropes, such as water/methanol/methyl chloroacetate, water/ethanol/ethyl acetate, water/n-propanol/n-propyl acetate, and water/isoamyl alcohol/isoamyl acetate.

Extensive literature data on azeotropic compositions and reliable empirical formulae for correction factors for specific physical conditions are available. On this basis, the minimum amount of additional azeotrope forming alcohol precursor required for attaining a total concentration of the alcohol in excess of its azeotropic concentration, can readily be calculated. Higher amounts may be added and may contribute to a higher degree of purification. Upper limits will essentially be determined by economic considerations.

It is preferred, that the reaction product to be recovered by the process of the invention is an ester of an unsaturated carboxylic acid, as such products find extensive use in polymerisation processes imposing severe standards on their impurity. Representative unsaturated carboxylic acids may have from 3 to 6 carbon atoms.

The azeotrope-forming alcohol precursor in the process of the invention preferably is an alkanol, typically having from 1 to 6, more typically from 1 to 4 carbon atoms. Consequently, the corresponding reaction product is an alkyl ester of an unsaturated carboxylic acid.

Most preferred reaction products are constituted by the class of C₁-C₄-alkyl acrylates and methacrylates, in which case the azeotrope-forming alcohol precursor is the corresponding C₁-C₄-alkanol. Examples of most preferred reaction products are methyl acrylate, ethyl acrylate, n-butyl acrylate, methyl methacrylate and n-butyl methacrylate.

A most preferred reaction product as methyl methacrylate may have been prepared by direct esterification of methacrylic acid with methanol in the presence of, for example, an acid catalyst, or by reaction of propyne with carbon monoxide and methanol in the presence of a cationic palladium/phosphine catalyst. As indicated above, the alcohol will normally have been present in the reaction zone in stoichiometric excess relative to methacrylic acid or propyne, respectively. Whether or not all methacrylic acid or propyne has been converted, the reaction effluent will then contain residual alcohol besides the methyl methacrylate product.

Reaction mixtures, from which carboxylate ester reaction products are to be recovered in accordance with the invention, may besides intermediate impurities comprise high and/or low boiling ends, it means impurities having a lower or higher volatility than the reaction product or azeotrope, respectively, or vice versa, if the azeotrope is a negative azeotrope. For obtaining a pure product, it is desired that such impurities are removed.

Therefore, according to a preferred embodiment of the invention, the process further comprises the step of separating a stream comprising heavy ends having a volatility lower than the volatilities of the reaction product and azeotrope. This tailing of heavy ends can be effected using common chemical technology, for example by distillation. The heavy ends may be discarded in a responsible manner. Frequently, the heavy ends will comprise catalyst or catalyst residues, in which case recycling of the catalyst or some of its components, optionally after regeneration, may be considered for economical reasons. The step of tailing the heavy ends may be effected prior to or after the separation of the azeotrope stream.

Another preferred embodiment of the process according to the invention further comprises the step of separating a stream comprising light ends having a volatility higher than the volatilities of the azeotrope and reaction product. The topping of light ends can be effected using common chemical technology, for example by distillation or flashing.

In the fractionations according to the invention, the several streams are differentiated and separated by their different volatilities. The fractionation may be effected by applying a temperature gradient under isobaric conditions. It may also be effected by staged pressure release under isothermic or pseudo-isothermic conditions. It is preferred that the fractionation is effected by distillation.

Taken as an example for illustrative purposes, the reaction effluent of the carbonylation of propyne in the presence of methanol (MeOH) to produce methyl methacrylate (MMA) may comprise 52 mol% of MMA and 46 mol% MeOH besides minor amounts of impurities, such as water and acetone. When the MMA product is to be recovered in accordance with the invention, the reaction effluent may be fractionated in a first distillation column having 16 trays applying a feed entry at tray No. 6, a pressure of 0.5 barabs and temperatures at the bottom and top of the column of 80 and 43 °C, respectively. This will result in a cut of the stream at slightly under the boiling point of pure MMA providing a heavy ends-containing MMA product stream over the bottom of the column, and over the top a first azeotrope stream comprising 9.34 mol% of MMA, 90.45 mol% of MeOH and 0.21 mol% of water, besides trace amounts of impurities more volatile than MMA. If desired, impurities boiling at lower temperatures than the azeotrope may be removed from the process at this point, for example by flashing or distillation. The azeotropic composition under the conditions applied is 4 mol% of MMA and 96 mol% of MeOH.

Direct tailing of this azeotrope stream for removal of trace impurities, such as acetone, boiling in the range between the azeotrope and pure MMA, and without applying the teaching of the present invention, by distillative fractionation over a second column (10 trays; feed entry: tray No. 6; p = 0.5 barabs; T_{bot} = 59 °C; Tₜₒₚ = 45 °C) will provide a top product for recycle comprising 7.59 mol% of MMA, 92.37 mol% of MeOH and 0.04 mol% of water, substantially free of intermediate impurities, and a bottom product for disposal comprising 57.59 mol% of MMA, 37.11 mol% of MeOH and 5.30 mol% of water, besides intermediate impurities being withdrawn from the process at this point.

In accordance with the invention, additional MeOH is added to this first azeotrope stream for raising its molar concentration to 94.60 mol%, whereby an alcohol enriched first azeotrope stream of composition of 5.28 mol% of MMA, 94.60 mol% of MeOH and 0.12 mol% of water, besides trace amounts of impurities is obtained. Fractionation of this stream over a second column (10 trays; feed entry: tray No. 6; p = 0.5 barabs; T_{bot} = 55 °C; Tₜₒₚ = 45 °C) provides a top product for recycle comprising 5.33 mol% of MMA, 94.66 mol% of MeOH and 0.01 mol% of water, substantially free of intermediate impurities, and a bottom product for disposal comprising 3.26 mol% of MMA, 91.72 mol% of MeOH and 5.00 mol% of water, besides the intermediate impurities.

It is seen that the invention effects a substantial reduction of the proportion of MMA discarded over the bottoms of the second column. Moreover, the high proportion of methanol in this bottoms stream obtained in accordance with the invention, precludes any miscibility problems regarding the amount of water present.

Expressed in terms of flow for a process generating a first azeotrope stream at a rate of 380.50 kg/hr of MMA, the molar composition data may be tabulated as follows:

| | Invention: | Outside invention: |
|---|---|---|
| First azeotrope | | |
| MMA | 380.5 kg/hr | 380.5 kg/hr |
| MeOH | 1179.9 kg/hr | 1179.9 kg/hr |
| water | 1.6 kg/hr | 1.6 kg/hr |
| | | |
| MeOH (added) | 1000 kg/hr | - |
| | | |

| Recycle stream | | |
|---|---|---|
| MMA | 375.2 kg/hr | 298.7 kg/hr |
| MeOH | 2132.1 kg/hr | 1163.0 kg/hr |
| water | 0.1 kg/hr | 0.2 kg/hr |
| | | |

| Disposal stream | | |
|---|---|---|
| MMA | 5.3 kg/hr | 81.8 kg/hr |
| MeOH | 47.8 kg/hr | 16.9 kg/hr |
| water | 1.5 kg/hr | 1.4 kg/hr |

## Claims

1. A process for recovery of a carboxylate ester reaction product from a reaction zone by withdrawal and fractionation of a reaction mixture comprising said carboxylate ester in admixture with an azeotrope-forming alcohol precursor as well as further impurities, which process comprises the steps of:
a) fractionating the reaction mixture into a carboxylate ester product-containing stream and a first azeotrope stream;
b) adding sufficiently additional azeotrope-forming alcohol precursor to the first azeotrope stream obtained by step (a) for providing a total concentration of the alcohol in excess of its azeotropic concentration; and
c) fractionating the alcohol-enriched stream obtained by step (b) into a second azeotrope stream for recycle to the reaction zone and a first residue stream for disposal.

2. A process as claimed in claim 1, further comprising the step of separating a stream comprising heavy ends having a volatility lower than the volatilities of the reaction product and azeotrope.

3. A process as claimed in claim 1 or 2, further comprising the step of separating a stream comprising light ends having a volatility higher than the volatilities of the azeotrope and reaction product.

4. A process as claimed in any one or more of claims 1-3, wherein the reaction product is an ester of an unsaturated carboxylic acid.

5. A process as claimed in claim 4, wherein the azeotrope-forming alcohol precursor is an alkanol.

6. A process as claimed in claim 5, wherein the reaction product is an alkyl acrylate or methacrylate and the azeotrope-forming alcohol precursor is the corresponding alkanol.

7. A process as claimed in any one or more of claims 1-6, wherein the fractionation is effected by distillation.

## Patentansprüche

1. Verfahren zur Isolierung von Carbonsäureesterreaktionsprodukten aus einer Reaktionszone durch Entnahme und Fraktionierung einer Reaktionsmischung, die diese Carbonsäureester in Mischung mit einer azeotropbildenden Alkoholvorstufe sowie mit weiteren Verunreinigungen enthält, umfassend folgende Schritte:
a) Fraktionierung der Reaktionsmischung in einen das Carbonsäureesterprodukt enthaltenden Strom und einen ersten Azeotropstrom,
b) Versetzen des in Schritt (a) erhaltenen ersten Azeotropstroms mit zusätzlicher azeotropbildender Alkoholvorstufe, die zur Erzielung einer über der azeotropen Konzentration liegenden Gesamtkonzentration an Alkohol ausreicht, sowie
c) Fraktionierung des in Schritt (b) erhaltenen alkoholangereicherten Stroms in einen zweiten zur Rückführung in die Reaktionszone bestimmten Azeotropstrom und in einen ersten zur Entsorgung bestimmten Rückstandsstrom.

2. Verfahren nach Anspruch 1, das als weiteren Schritt die Abtrennung eines Stroms aus schwerflüchtigen Anteilen mit einer geringeren Flüchtigkeit als der des Reaktionsprodukts und der des Azeotrops aufweist.

3. Verfahren nach Anspruch 1 oder 2, das als weiteren Schritt die Abtrennung eines Stroms aus leichtflüchtigen Anteilen mit einer höheren Flüchtigkeit als der des Reaktionsprodukts und der des Azeotrops aufweist.

4. Verfahren nach einem oder mehreren der Ansprüche 1-3, wobei das Reaktionsprodukt ein Ester einer ungesättigten Carbonsäure ist.

5. Verfahren nach Anspruch 4, wobei die azeotropbildende Alkoholvorstufe ein Alkanol ist.

6. Verfahren nach Anspruch 5, wobei das Reaktionsprodukt ein Alkylacrylat bzw. -methacrylat und die azeotropbildende Alkoholvorstufe das entsprechende Alkanol ist.

7. Verfahren nach einem oder mehreren der Ansprüche 1-6, wobei die Fraktionierung destillativ erfolgt.

## Revendications

1. Procédé de récupération d'un produit de réaction d'un ester du type carboxylate à partir d'une zone de réaction par prélèvement et fractionnement d'un mélange réactionnel comprenant ledit ester du type carboxylate en mélange à au moins un précurseur d'alcool formateur d'azéotrope, comme aussi d'autres impuretés, caractérisé en ce qu'il comprend les étapes consistant à :
a) fractionner le mélange réactionnel en un courant contenant l'ester du type carboxylate produit et un premier courant d'azéotrope;
b) ajouter suffisamment de précurseurs du type alcool formateur d'azéotrope supplémentaire au premier courant d'azéotrope obtenu à l'étape a) pour assurer une concentration totale de l'alcool supérieure à sa concentration azéotropique, et
c) fractionner le courant enrichi en alcool obtenu dans l'étape b) en un second courant d'azéotrope destiné à être recyclé dans la zone de réaction et un premier courant de résidu à jeter.

2. Procédé suivant la revendication 1, caractérisé en ce qu'il comprend, en outre, l'étape consistant à séparer un courant comprenant des extrémités lourdes possédant une volatilité inférieure aux volatilités du produit de réaction et de l'azéotrope.

3. Procédé suivant la revendication 1 ou 2, caractérisé en ce qu'il comprend, en outre, l'étape consistant à séparer un courant comprenant des extrémités légères possédant une volatilité supérieure aux volatilités de l'azéotrope et du produit de réaction.

4. Procédé suivant l'une quelconque des revendications 1 à 3, caractérisé en ce que le produit de réaction est un ester d'un acide carboxylique insaturé.

5. Procédé suivant la revendication 4, caractérisé en ce que le précurseur du type alcool formateur d'azéotrope est un alcanol.

6. Procédé suivant la revendication 5, caractérisé en ce que le produit de réaction est un acrylate d'alkyle ou un méthacrylate d'alkyle et le précurseur de type alcool formateur d'azéotrope est l'alcanol correspondant.

7. Procédé suivant une ou plusieurs des revendications 1 à 6, caractérisé en ce que l'on entreprend le fractionnement par distillation.
